(19) Europäisches Patentamt
European Patent Office
Office européen des brevets

(11) **EP 2 962 091 B1**

(12) **EUROPÄISCHE PATENTSCHRIFT**

(45) Veröffentlichungstag und Bekanntmachung des Hinweises auf die Patenterteilung:
**22.02.2017 Patentblatt 2017/08**

(21) Anmeldenummer: **14708229.1**

(22) Anmeldetag: **28.02.2014**

(51) Int Cl.:
***G01N 27/22*** *(2006.01)* ***G01N 33/28*** *(2006.01)*

(86) Internationale Anmeldenummer:
**PCT/EP2014/053940**

(87) Internationale Veröffentlichungsnummer:
**WO 2014/131885 (04.09.2014 Gazette 2014/36)**

(54) **ANORDNUNG ZUR BESTIMMUNG DER PHASENVERTEILUNG IN MEHRPHASIGEN MEDIEN MIT MINDESTENS EINER HOCHLEITFÄHIGEN PHASE**

ARRANGEMENT FOR DETERMINING THE PHASE DISTRIBUTION IN MULTIPHASE MEDIA HAVING AT LEAST ONE HIGHLY CONDUCTIVE PHASE

SYSTÈME DE DÉTERMINATION DE LA DISTRIBUTION DES PHASES DE MILIEUX POLYPHASIQUES COMPRENANT AU MOINS UNE PHASE HAUTEMENT CONDUCTRICE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.02.2013 DE 102013203437**

(43) Veröffentlichungstag der Anmeldung:
**06.01.2016 Patentblatt 2016/01**

(73) Patentinhaber: **Helmholtz-Zentrum Dresden - Rossendorf e.V.**
**01328 Dresden (DE)**

(72) Erfinder:
• **SCHLEICHER, Eckhard**
**01219 Dresden (DE)**
• **LÖSCHAU, Martin**
**02625 Bautzen (DE)**
• **VAN CAMPEN, Laurens**
**NL-2628 ED Delft (NL)**

(74) Vertreter: **Kailuweit & Uhlemann Patentanwälte Partnerschaft mbB**
**Bamberger Straße 49**
**01187 Dresden (DE)**

(56) Entgegenhaltungen:
DE-A1- 19 649 011     DE-A1-102006 019 178
US-A- 4 644 263      US-A- 5 210 499

## Beschreibung

## Technische Gebiet

Die Erfindung betrifft eine Anordnung zur Messung der Phasenverteilung eines mehrphasigen Stoffgemisches mit gasförmigen und flüssigen Komponenten unter Vorhandensein einer hochleitfähigen Phase. Unter hochleitfähiger Phase werden im Folgenden Medien mit hoher Leitfähigkeit, beispielsweise Salzwasser oder Flüssigmetalle verstanden.

Anwendungsgebiete der vorliegenden Erfindung sind beispielsweise die Bestimmung der Flüssigkeitsverteilung und des Füllstands in Gefäßen, sowie die Untersuchung von Gas-Flüssigkeits-Mehrphasenströmungen, insbesondere in Rohrleitungen z.B. in der Erdölproduktion und Verarbeitung.

## Stand der Technik

Für die Untersuchung von Zweiphasenströmungen oder von Flüssigkeitsverteilungen in Rohrleitungen und Gefäßen werden häufig Gittersensoren eingesetzt. In US 4 644 263 A, US 5 210 499 A und DE 19 649 011 C2 werden Anordnungen beschrieben, mit denen die elektrische Leitfähigkeit innerhalb eines Messquerschnitts mit Hilfe einer gitterförmigen Elektrodenanordnung und zugeordneter Elektronik gemessen werden kann. Bei diesen Anordnungen werden drahtförmige Elektroden einer Anregungselektrodenebene des Gitters, die mit dem Medium in elektrisch leitfähiger Verbindung stehen, nacheinander mit einem Spannungssignal beaufschlagt. An den Drahtelektroden einer parallel hierzu in geringem Abstand um einen ebenen Winkel verdreht angeordneten Empfängerelektrodenebene des Gitters wird ein Stromsignal erfasst. Dadurch sind diese Anordnungen in der Lage, die Leitfähigkeit zwischen beiden Ebenen in den Kreuzungspunkten der Projektionen der Elektroden (im Folgenden als "Kreuzungspunkte" bezeichnet) mit sehr hoher Messfrequenz zu bestimmen.

Für eine Zweiphasenströmung mit genau einer leitfähigen Phase, beispielsweise ein Gas-Wasser-Gemisch, kann die Phasenverteilung im Strömungsquerschnitt durch Erfassung der Leitfähigkeitsverteilung bestimmt werden. Eine Phasendiskriminierung für ähnlich gut oder schlecht leitende Phasen oder Komponenten einer Strömung ist mit diesen Anordnungen nicht direkt möglich.

In DE 10 2007 019 926 B4 wird ein Gittersensor beschrieben, welcher durch die Messung der komplexen elektrischen Admittanz im Messquerschnitt auch in der Lage ist nichtleitfähige Komponenten im Messquerschnitt von einander zu differenzieren und deren Anteil zu bestimmen.

DE 101 36 358 A1 schlägt zur Leitfähigkeitsmessung eines einheitlichen Fluids die Verwendung eines Kanals vor. Der Einsatz zur Bestimmung der Leitfähigkeit vermischter Fluide, ist nicht vorgesehen. Es ist damit nur der Einsatz in Batch-Anlagen möglich, wenn unterschiedliche, nicht gemischte, Fluide zeitlich hintereinander durch den Kanal fließen.

DE 10 2006 019 178 A1 beschreibt einen Gittersensor, der zur Messung von nichtleitenden Komponenten, durch Erfassung der komplexwertigen elektrischen Admittanz, geeignet ist.

DE 10 2005 019 739 B3 beschreibt einen Gittersensor, der bei wechselnden Temperaturen und Drücken eingesetzt werden kann, in dem die Drähte der Elektrodenebenen nicht starr, sondern derart im Sensorkörper befestigt sind, dass sie sich bei Änderungen der Temperatur- und/oder des Drucks ausdehnen können.

Die bisher bekannten Lösungsansätze können für hochleitfähige Medien jedoch nicht eingesetzt werden, da in solchen Fällen die Leitfähigkeit wenigstens einer Komponente der Strömung in die Nähe der Leitfähigkeit der Drähte des Sensors kommt. Dadurch werden alle vom Medium benetzten Drähte quasi kurzgeschlossen und somit können die einzelnen Kreuzungspunkte nicht mehr unabhängig voneinander ausgelesen werden.

Eine Alternative zur Bestimmung der kompletten Phasenverteilung in einer Messebene ist die Röntgentomographie. Viele unterschiedliche Röntgenpfade müssen in diesem Fall aufwendig rekonstruiert werden. Die Röntgentomographie hat den Vorteil, dass sie in den Prozess nicht störend eingreift, weil sie außerhalb des Prozesses angeordnet sein kann, aber erfordert einen großen Aufwand, damit Mitarbeiter vor Strahlung geschützt werden und wird deshalb in industriellen Anwendungen eher abgelehnt.

## Beschreibung der Erfindung

### Technische Aufgabe

Aufgabe der vorliegenden Erfindung ist es, eine Anordnung zur schnellen Messung der Phasen- oder der Komponentenverteilung in einem Strömungsquerschnitt für Stoffgemische mit sowohl nichtleitfähigen Komponenten, z. B. Öl oder Gas und/oder leitfähigen Komponenten, sowie hochleitfähigen Komponenten, z. B. Salzwasser, anzugeben. Unter schneller Messung werden Messungen mit einer diskreten Zeitdifferenz kleiner als 1 ms, bevorzugt ca. 100 µs oder kleiner verstanden.

### Technische Lösung der Erfindung

Die Lösung der Aufgabe erfolgt mit der Anordnung gemäß Anspruch 1. Vorteilhafte Ausführungen sind in den rückbezogenen Unteransprüchen angegeben.

Die Messung der Phasenverteilung erfolgt über Eigenschaften, die die verschiedenen Phasen unterscheiden. Da viele mehrphasige Strömungen intransparent sind, ist der Einsatz von sichtbaren Licht in der Regel nicht geeignet, ausgenommen punktuell messende Nadelsonden zur lokalen Blasen-Identifikation.

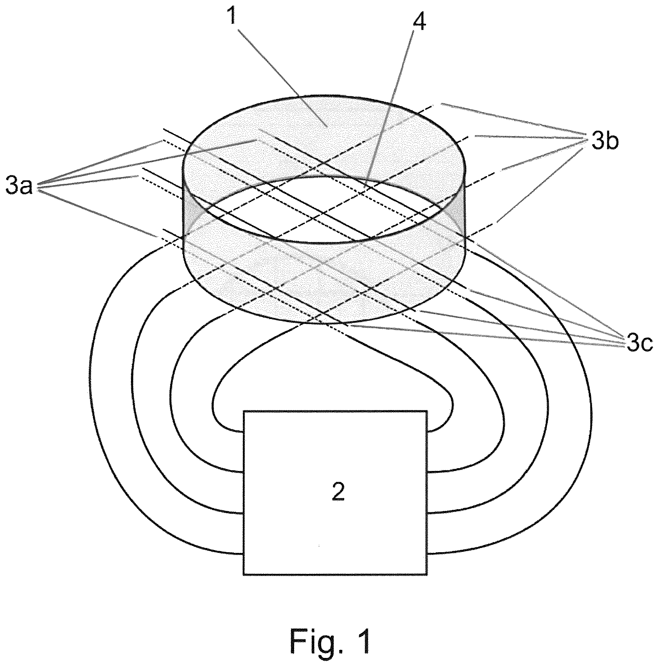

Die erfindungsgemäße Anordnung (Fig. 1) umfasst einen Gittersensor (1) mit mindestens drei in geringem Abstand zueinander befindlichen Elektrodenebenen und einer zugeordneten Messelektronik (2). Der Gittersensor besitzt drei Ebenen drahtförmiger Elektroden, die als Sendeelektroden (3a), Empfängerelektroden (3b) sowie Erdungselektroden (3c) bezeichnet sind und die innerhalb jeder Ebene in einem geringen Abstand parallel zueinander angeordnet sind. Jeder Ebene ist damit genau eine Aufgabe zugeordnet.

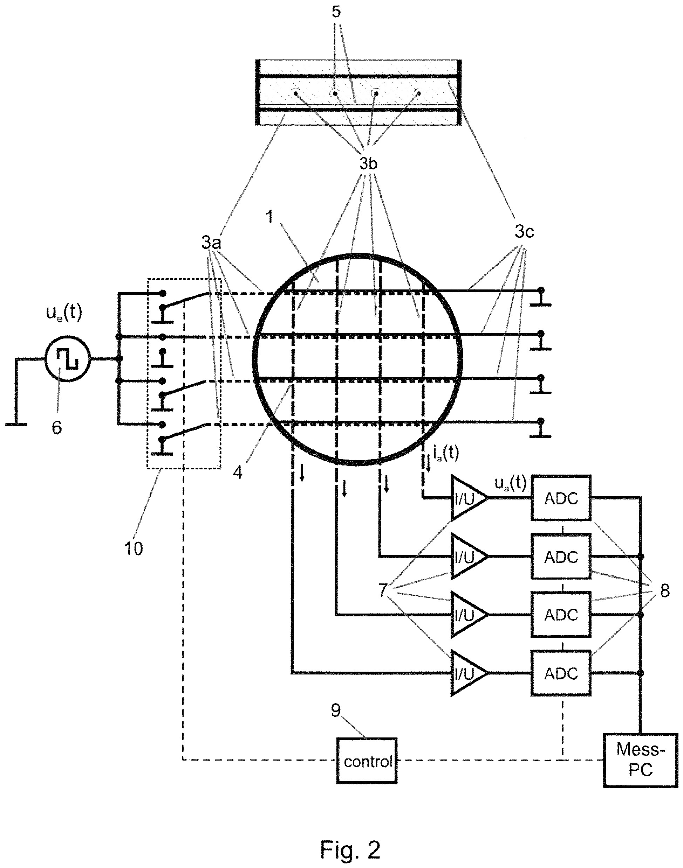

Die Orientierung der Elektroden verschiedener Ebenen zueinander ist in einem Winkel zueinander verdreht, so dass Kreuzungspunkte zwischen der Ebene der Sendeelektroden und der Empfängerelektroden gebildet werden. Vorzugsweise ist diese Verdrehung orthogonal. Gemessen wird die elektrische Kapazität (oder Permittivität) des Mediums zwischen den Sendeelektroden (3a) und den Empfängerelektroden (3b) in jedem einzelnen Kreuzungspunkt (4) des Elektrodengitters. Hierfür (Fig. 2) werden die Sendeelektroden (3a) und die Empfängerelektroden (3b) mit einer elektrisch isolierenden Schicht (5) überzogen, die eine galvanische Trennung zum Medium ermöglicht, während die Erdungsdrähte (3c) blank bleiben um das gesamte Fluid auf Massepotential zu halten.

Zur Messung der elektrische Kapazität (oder Permittivität) (Fig. 2) des Mediums im einzelnen Kreuzungspunkt (4) wird die zugehörige Sendeelektrode (3a) mittels eines Frequenzgenerators (6) mit einer Wechselspannung beaufschlagt, während alle anderen Sendeelektroden auf Masse geschaltet werden. Gleichzeitig wird an allen Empfängerelektroden (3b) parallel die Sprungantwortfunktion des durch das Untersuchungsmedium im Kreuzungspunkt (4) fließenden kapazitiven Verschiebestroms mit Hilfe eines an die Empfängerelektrode angeschlossenen Strom-Spannungswandlers (7) erfasst und in ein äquivalentes Spannungssignal gewandelt. Als Wechselspannung kommen bevorzugt rechteck- oder trapezförmigen Wechselspannungen zum Einsatz, weil sich damit die Messergebnisse am besten auswerten lassen.

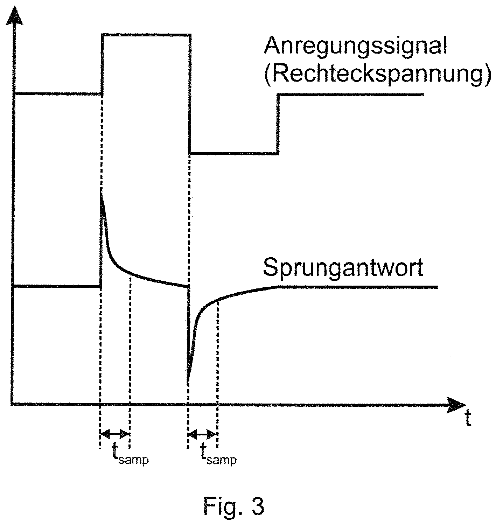

Die Sprungantwort (Fig. 3) des Systems auf die Flanke des Anregungssignals hängt vom Verstärkungs-Bandbreiten-Produkt des Strom-Spannungswandlers (7), der Rückkoppelimpedanz sowie der elektrischen Permittivität des Mediums im Kreuzungspunkt (4) ab. Bei konstanten Verstärkungs- und Geometriebedingungen kann somit aus der Sprungantwort auf die Permittivität im Kreuzungspunkt geschlossen werden. Hierfür wird zu einem oder mehreren bestimmten Zeitpunkten $t_{samp}$ nach erfolgter Flanke mittels eines Analog-DigitalWandlers (8) die Ausgangsspannung des Strom-Spannungswandlers (7) digitalisiert und elektronisch erfasst. Zur zeitlichen Synchronisation von Anregungsimpuls und Analog-Digital-Umwandlung dient eine Steuereinheit (9) z. B. ein Mikrokontroller.

_Vorteile der Erfindung_

Neuartig an der Erfindung ist die Möglichkeit einer schnellen zweidimensionalen Messung der Verteilung von nichtleitfähigen, sowie leitfähigen und/oder hoch leitfähigen Komponenten im Querschnitt einer Mehrphasenströmung, welche erstmals die Vermessung von Phasenanteilen und -Verteilungen in Salzwasser-Öl-Gas-Gemischen ermöglicht.

_Anwendungsbereiche der Erfindung_

In industriellen Anlagen bestehen Strömungen häufig aus mehr als einer Phase, wie z. B. in der Ölproduktion, wo Strömungsgemische aus Öl, Gas, Wasser und Sand auftreten können, in Chemieanlagen, wo Vermischungs- und Trennungsvorgänge auftreten, oder in der Stahlindustrie (flüssiger Stahl/Schlacke/Luft). Um die Vermischung bzw. Trennung der verschiedenen Phasen zu studieren oder überprüfen zu können, müssen diese verschiedenen Phasen gemessen und bestimmt werden.

Die relative Permittivität unterscheidet sich in der Regel zwischen verschiedenen Medien und damit den Phasen einer Gemisch-Strömung. Das Messprinzip des Gittersensors nutzt diese physikalische Eigenschaft aus, um zweidimensionale Bilder der Phasenverteilung im Mess-Querschnitt mit hoher räumlicher und zeitlicher Auflösung zu generieren. Diese Methode wurde zunächst mit einer quasi DC-Anregung zur Messung von Gasen in leitfähigen Medien eingesetzt. Für nicht-leitfähige Medien kann die Dielektrizitätskonstante durch AC-Anregung und Messung der Phasenverschiebung und Amplitudendämpfung bestimmt werden.

Bei Medien mit einer sehr niedrigen Impedanz, d.h. hohen Leitfähigkeit, deren Leitfähigkeit in den Bereich der Elektroden im Sensorgitter kommt (wie z. B. Salzwasser), werden die Messungen durch quasi Kurzschluss aller Drähte im Kontakt mit dem hoch leitfähigen Medium verzerrt, wenn keine Erdungsebene verwendet wird. Der Gittersensor mit Erdungsebene löst dieses Problem durch die Erdung des kompletten Flüssigkeitsquerschnittes und der Isolation der Sende- und Empfängerdrähte.

Viele Anwendungen mit mehrphasigen Strömungen enthalten hoch leitfähige Flüssigkeiten. In der Förderung von Erdöl wird häufig Sole (Salzwasser) gemeinsam mit Öl vorgefunden und gefördert. Die Phasenverteilung in einem Rohr mit Öl und Salzwasser kann daher weder mit einem konventionellen Leitfähigkeitsnoch mit einem kapazitiv messenden Gittersensor ermittelt werden. Die erfindungsgemäße Anordnung erlaubt hingegen die Messung der Phasenverteilung in solchen Fällen.

**Ausführungsbeispiele**

Die Ausführungsbeispiele der Erfindung werden mit Zeichnungen beschrieben.

Die Zeichnungen Fig. 1, Fig. 2 zeigen schematisch als ein Ausführungsbeispiel Gittersensoranordnungen mit 4 Sende-, 4 Empfänger- und 4 Erdungselektroden und einer Rundgeometrie. Die Gittersensoren können auch in anderen Geometrien, z. B. rechteckigen Querschnitten, aufgebaut sein. Weiterhin ist die Anzahl von Elektroden theoretisch beliebig.

Die Zeichnung Fig. 1 zeigt schematisch einen Gittersensor (1) mit drei Elektrodenebenen und eine dazugehörige Messelektronik (2). Der Gittersensor verfügt über je vier Metalldrähte pro Ebene (3a - Sendeelektroden, 3b - Empfängerelektroden, 3c - Erdungselektroden), welche elektrisch voneinander isoliert über den Sensorquerschnitt gespannt sind. Die Verankerung der Drähte im Sensorrahmen erfolgt derartig, dass jede Sende- und Empfängerelektrode vollständig von den anderen Elektroden sowie dem Rahmen selbst elektrisch isoliert ist.

Die Messelektronik (Fig. 2) besteht auf der Anregungsseite aus einem Rechteckgenerator (6), einem Multiplexer (10) und einem Kontroller (9). Die einzelnen Sendeelektroden (3a) der Sendeebene des Sensors sind elektrisch mit den Ausgängen des Multiplexers (10) verbunden. Auf der Empfängerseite ist jede der Empfängerelektroden (3b) der Empfängerebene mit einem Strom-Spannungswandler (7) verbunden. An die Strom-Spannungswandler (7) sind zur Aufzeichnung der Sprungantwort Analog-Digital-Umwandler (8) angeschlossen, welche taktsynchron mit Hilfe einer Steuereinheit (9) zu einem definierten Zeitpunkt $t_{samp}$ nach erfolgter Anregungsflanke den aktuellen Wert des Spannungsverlaufs am Strom-Spannungs-Wandler (7) erfassen.

Das Messschema des in Fig. 1 und Fig. 2 skizzierten Sensors ist wie folgt: Durch einen für die Steuerung vorgesehenen Kontroller oder Mikroprozessor (9) wird das rechteck- oder trapezförmige Spannungssignal des Frequenzgenerators (6) nacheinander auf die einzelnen Sendeelektroden (3a) über den Multiplexer (10) aufgeschaltet. Der Multiplexer (10) ist so ausgeführt, dass nur eine einzelne Sendeelektrode (3a) mit der Rechteck- oder Trapezspannung beaufschlagt wird, während alle anderen Sendeelektroden auf Nullpotential liegen. An der jeweils aktiven Sendeelektrode fließt in den virtuellen Kreuzungspunkten (4) der Drahtelektroden ein kapazitiver Verschiebestrom zu den auf virtueller Masse liegenden Empfängerelektroden (3b). Ein DC-Stromfluss ist auf Grund der isolierenden Beschichtung (5) (z. B. durch Isolierlack oder Isolierschlauch) an den Sendeelektroden (3a) und Empfängerelektroden (3b) ausgeschlossen. Nach erfolgtem Spannungssprung an der Anregungselektrode (3a) folgt der Stromfluss an der Empfängerelektrode (3b) als kapazitiver Verschiebestrom dem Anregungsstrom in der Sendeelektrode (3a) mit der Exponentialfunktion

$$i(t) = -\frac{U_0}{R_V} \cdot e^{-\frac{t}{\tau}}$$

mit

$$\tau = R_V \cdot C.$$

Wobei $U_0$ die Amplitude der Anregungsspannung, $R_V$ der Vorwiderstand (Summe aus Leitungswiderständen und Drahtwiderstand) und $C$ die Kapazität im Kreuzungspunkt sind. Die Kapazität $C$ wiederum hängt lediglich von der elektrischen Permittivität im Kreuzungspunkt ($\varepsilon_{rel}$) ab, da die Geometrie der virtuellen Kreuzungspunkte (4) als konstant angenommen werden kann.

Damit gilt:

$$\ln i(t) \sim \frac{1}{\varepsilon_{rel}}.$$

Da der Strom über den Strom-Spannungs-Wandler (7) linear in eine äquivalente Spannung gewandelt wird, kann die gemessene Spannung auch als indirekt proportional zur Dielektrizitätskonstante des Mediums im Kreuzungspunkt betrachtet werden. Der Vorteil des exponentiellen Zusammenhangs ist, dass selbst kleine Änderungen z. B. Luft ($\varepsilon_{rel}$ = 1) und Öl ($\varepsilon_{rel}$ = 2 bis 3) gut voneinander zu unterscheiden sind, selbst im Beisein von Wasser ($\varepsilon_{rel}$ = 80) ohne im Dynamikbereich unterzugehen. Die vorliegende Anordnung ist auch in der Lage, die relative Permittivität $\varepsilon_{rel}$ zu bestimmen. Dem Fachmann ist geläufig, dass dafür eine Kalibrierung mit einem bekannten Medium, wie etwa Wasser oder Luft, benötigt wird. Aus dieser Kalibrierung wird der Geometriefaktor bestimmt. Folglich kann damit aus der Messung die relative Permittivität $\varepsilon_{rel}$ bestimmt werden.

**Bezugszeichenliste**

1 - Gittersensor
2 - Messelektronik
3a - Sendeelektroden - mit Punkt-Punkt-Linie in Fig. 1 oder Fig. 2 dargestellt
3b - Empfängerelektroden - mit Strich-Strich-Linie in Fig. 1 oder Fig. 2 dargestellt
3c - Erdungselektroden
4 - Kreuzungspunkt
5 - isolierende Schicht
6 - Frequenzgenerator
7 - Strom-Spannungswandler
8 - Analog-Digital-Wandler
9 - Steuereinheit

10 - Multiplexer

**Patentansprüche**

1. Anordnung zur Bestimmung der Phasenverteilung in mehrphasigen Fluiden mit mindestens einer hochleitfähigen Phase bei weiterem Vorhandensein von nichtleitfähigen und/oder anderen leitfähigen Komponenten, umfassend drei übereinander liegende Elektrodenebenen von drahtförmigen Elektroden, welche in einem Sensorrahmen aufgespannt sind,

    a. wobei die Elektroden in jeder Ebene parallel mit einem geringen Abstand zu einander angeordnet sind,
    b. wobei zwei der Elektrodenebenen durch eine isolierende Schicht (5) galvanisch vom Untersuchungsmedium getrennt sind und eine dieser beiden Elektrodenebenen als Senderebene (3a) und eine andere als Empfängerebene (3b) fungiert, und die Orientierung der Elektroden in diesen beiden Ebenen in einem Winkel zueinander verdreht ist, wobei die Ebenen parallel zueinander angeordnet sind,
    c. **dadurch gekennzeichnet, dass** die dritte Elektrodenebene (3c) hingegen nicht isoliert ist und auf Erdpotential liegt und somit mit dieser in Kontakt stehende hochleitfähige Phasenanteile ebenfalls auf Erdpotential liegen und
    d. wobei die Anordnung mit einer elektronischen Messeinrichtung verbunden ist, um die elektrische Kapazität oder Permittivität des Mediums in den einzelnen Kreuzungspunkten (4), die von den Sendeelektroden (3a) und den Empfängerelektroden (3b) gebildet werden, zu messen, und wobei die elektronische Messeinrichtung ausgebildet ist, nacheinander die zugehörigen Sendeelektroden (3a) mit einer Wechselspannung zu beaufschlagen, und währenddessen alle anderen Sendeelektroden (3a) auf Masse zu schalten und gleichzeitig an allen Empfängerelektroden (3b) parallel die Sprungantwortfunktion des Stromsignals zu messen und daraus auf die Permittivität des Untersuchungsmediums zu schließen.

2. Anordnung nach Anspruch 1, **dadurch gekennzeichnet, dass** die Orientierung der Elektroden der Senderebene (3a) und der Empfängerebene (3b) zueinander orthogonal ist.

3. Anordnung nach einem der vorhergehenden Ansprüche, **dadurch gekennzeichnet, dass** die isolierten Elektroden der Sende- und/oder Empfängerebene zur Isolierung mittels eines Lackes oder eines Kunststoffes beschichtet sind oder mit einem Isolierschlauch oder Isolierröhrchen isoliert sind.

4. Anordnung nach einem der vorhergehenden Ansprüche, **dadurch gekennzeichnet, dass** die Messeinrichtung einen Frequenzgenerator enthält.

5. Anordnung nach einem der vorhergehenden Ansprüche, **dadurch gekennzeichnet, dass** die Messeinrichtung einen Multiplexer enthält, der das nacheinander Aufschalten des Anregungssignals realisiert.

6. Anordnung nach einem der vorhergehenden Ansprüche, **dadurch gekennzeichnet, dass** die Messeinrichtung eine Steuereinheit (9) enthält, die den Anregungsimpuls und die Analog-Digital-Umwandlung zeitlich synchronisiert.

7. Anordnung nach einem der vorhergehenden Ansprüche, **dadurch gekennzeichnet, dass** die Messeinrichtung einen Strom-Spannungswandler enthält, der an die Empfängerelektroden angeschlossen ist, um die Sprungantwort des Stromsignals zu messen und in ein auswertbares Spannungssignal zu wandeln.

8. Anordnung nach Anspruch 7, wobei das Spannungssignal mittels eines Analog- DigitalWandlers digitalisiert werden kann.

9. Verfahren zur Bestimmung der Phasenverteilung in mehrphasigen Medien mit der Anordnung gemäß einem der vorhergehenden Ansprüche, **dadurch gekennzeichnet, dass** die elektrische Kapazität oder Permittivität des Mediums in einzelnen Kreuzungspunkt (4) nacheinander gemessen wird und dazu die zugehörigen Sendeelektroden (3a) mit einer Wechselspannung beaufschlagt werden, während alle anderen Sendeelektroden (3a) auf Masse geschaltet werden und gleichzeitig an allen Empfängerelektroden (3b) parallel die Sprungantwortfunktion des Stromsignals gemessen und daraus auf die Permittivität des Mediums geschlossen wird.

10. Verfahren nach Anspruch 9, **dadurch gekennzeichnet, dass** die verwendete Wechselspannung eine Rechteck- oder trapezförmige Wechselspannung ist.

**Claims**

1. An arrangement for determining the phase distribution in multiphase fluids having at least one highly conductive phase with the additional presence of nonconductive components and/or other conductive components, comprising three electrode levels of wire-type electrodes situated one above the other and spread out in a sensor frame,

a. wherein the electrodes in each level are arranged in parallel at a slight distance from one another,

b. wherein two of the electrode levels are separated galvanically from the test medium by an insulating layer (5), and one of these two electrode levels functions as a transmitter level (3a) and another electrode level functions as a receiver level (3b), and the orientation of the electrodes in these two levels is rotated at an angle to the other, wherein the levels are arranged parallel to one another,

c. **characterized in that**, however, the third electrode level (3c) is not insulated and is at ground potential and therefore is also at ground potential with these highly conductive phase components that are in contact, and

d. wherein the arrangement is connected to an electronic measuring device to measure the electrical capacitance or permittivity of the medium in the individual points of intersection (4) formed by the transmitter electrodes (3a) and the receiver electrodes (3b), and wherein the electronic measuring device is designed to apply an alternating voltage to the respective transmitter electrodes (3a) one after the other in succession, and meanwhile to switch all the other transmitter electrodes (3a) to ground and to measure the step function response of the current signal simultaneously on all receiver electrodes (3b) in parallel and to determine from this the permittivity of the test medium.

2. The arrangement according to claim 1, **characterized in that** the orientation of the electrodes of the transmitter level (3a) and of the receiver level (3b) is orthogonal to one another.

3. The arrangement according to any one of the preceding claims, **characterized in that** the insulated electrodes of the transmitter and/or receiver levels are coated with a paint or a plastic for insulation or are insulated by insulating tubes or an insulating tubing.

4. The arrangement according to any one of the preceding claims, **characterized in that** the measuring device includes a frequency generator.

5. The arrangement according to any one of the preceding claims, **characterized in that** the measuring device includes a multiplexer, which implements the sequential connection of the excitation signal.

6. The arrangement according to any one of the preceding claims, **characterized in that** the measuring device includes a control unit (9), which synchronizes the excitation pulse and the analog-digital conversion in time.

7. The arrangement according to any one of the preceding claims, **characterized in that** the measuring device includes a current-voltage converter, which is connected to the receiver electrodes to measure the step response of the current signal and convert it to a voltage signal that can be analyzed.

8. The arrangement according to claim 7, wherein the voltage signal can be digitized by means of an analog-digital converter.

9. A method for determining the phase distribution in multiphase media with an arrangement according to any one of the preceding claims, **characterized in that** the electrical capacitance or permittivity of the medium at individual points of intersection (4) is measured successively and, to do so, an alternating voltage is applied to the respective transmitter electrodes (3a), while all other transmitting electrodes (3a) are connected to ground, and the step function response of the current signal is measured in parallel on all receiver electrodes (3b) at the same time, and the permittivity of the medium is deduced from this.

10. The method according to claim 9, **characterized in that** the alternating voltage used is a square-wave alternating voltage or a trapezoidal alternating voyage.

**Revendications**

1. Agencement destiné à déterminer la distribution des phases dans des fluides polyphasés avec au moins une phase hautement conductrice, en présence par ailleurs de composants non conducteurs et/ou d'autres composants conducteurs, comprenant trois niveaux superposés d'électrodes en forme de fils métalliques, lesquelles sont tendues dans un cadre de capteurs,

a. dans chaque niveau, les électrodes étant placées en parallèle, avec un faible écart mutuel,

b. deux niveaux d'électrodes étant séparés de manière galvanique par une couche isolante (5) du milieu qui doit être analysé et l'un desdits deux niveaux d'électrodes faisant office de niveau émetteur (3a) et un autre de niveau récepteur (3b) et l'orientation des électrodes dans lesdits deux niveaux étant avec un angle réciproque, les niveaux étant placés à la parallèle l'un de l'autre,

c. **caractérisé en ce qu'**en revanche, le troisième niveau d'électrodes (3c) n'est pas isolé et est mis à un potentiel terrestre et de ce fait les parts de phase hautement conductrices qui sont en contact avec celui-ci sont également mises à un potentiel terrestre et

d. l'agencement étant connecté sur un système de mesure électronique pour mesurer la capacité ou permittivité électrique du milieu sur chacun des points d'intersection (4) qui sont créés par les électrodes émettrices (3a) et les électrodes réceptrices (3b) et le système de mesure électronique étant conçu pour appliquer successivement aux électrodes émettrices (3a) correspondantes une tension alternative et pendant ce temps, connecter toutes les autres électrodes émettrices (3a) à la masse et simultanément, mesurer à la parallèle de toutes les électrodes réceptrices (3b) la fonction de réponse transitoire du signal de courant et en déduire la permittivité du milieu qui doit être analysé.

2. Agencement selon la revendication 1, **caractérisé en ce que** l'orientation des électrodes du niveau émetteur (3a) et du niveau récepteur (3b) est orthogonale, l'une par rapport à l'autre.

3. Agencement selon l'une quelconque des revendications précédentes, **caractérisé en ce que** pour l'isolation, les électrodes isolées du niveau émetteur et/ou récepteur sont revêtues d'une laque ou d'une matière plastique ou sont isolées avec un flexible isolant ou un petit tube isolant.

4. Agencement selon l'une quelconque des revendications précédentes, **caractérisé en ce que** le système de mesure contient un générateur de fréquence.

5. Agencement selon l'une quelconque des revendications précédentes, **caractérisé en ce que** le système de mesure contient un multiplexeur qui réalise l'application successive du signal d'excitation.

6. Agencement selon l'une quelconque des revendications précédentes, **caractérisé en ce que** le système de mesure contient une unité de commande (9) qui synchronise dans le temps le signal d'excitation et le signal de conversion analogique-numérique.

7. Agencement selon l'une quelconque des revendications précédentes, **caractérisé en ce que** le système de mesure contient un transformateur de tension électrique qui est raccordé sur les électrodes réceptrices pour mesurer la réponse transitoire du signal de courant et la convertir en un signal de tension évaluable.

8. Agencement selon la revendication 7, le signal de tension pouvant être numérisé à l'aide d'un convertisseur analogique-numérique.

9. Procédé destiné à déterminer la distribution des phases dans des milieux polyphasés avec l'agencement selon l'une quelconque des revendications précédentes, **caractérisé en ce qu'**on mesure successivement la capacité ou permittivité électrique sur des points d'intersection (4) individuels et à cet effet, on applique une tension alternative sur les électrodes émettrices (3a) correspondantes, pendant qu'on connecte à la masse toutes les autres électrodes émettrices (3a) et simultanément, on mesure à la parallèle sur toutes les électrodes réceptrices (3b) la fonction de réponse transitoire du signal de courant et on en déduit la permittivité du milieu.

10. Procédé selon la revendication 9, **caractérisé en ce que** la tension alternative utilisée est une tension alternative rectangulaire ou trapézoïdale.

Fig. 1

Fig. 2

Anregungssignal
(Rechteckspannung)

Sprungantwort

$t_{samp}$          $t_{samp}$

t

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4644263 A
- US 5210499 A
- DE 19649011 C2
- DE 102007019926 B4
- DE 10136358 A1
- DE 102006019178 A1
- DE 102005019739 B3